Europäisches Patentamt

European Patent Office

Office européen des brevets

(19)

(11) Publication number: **0 395 519**
**A1**

# EUROPEAN PATENT APPLICATION

(21) Application number: **90401136.8**

(22) Date of filing: **25.04.90**

(51) Int. Cl.5: **A61B 5/022**

(30) Priority: **28.04.89 JP 111769/89**

(43) Date of publication of application:
**31.10.90 Bulletin 90/44**

(84) Designated Contracting States:
**DE FR GB**

(71) Applicant: **SONY CORPORATION**
**7-35, Kitashinagawa 6-chome Shinagawa-ku Tokyo(JP)**

(72) Inventor: **Takashima, Mitsuru, c/o Sony Corporation**
**7-35, Kitashinagawa 6-chome Shinagawa-ku, Toyko(JP)**
Inventor: **Woo, Beak Sang**
**A-302, Junone Villa, 925-1, Jeongneng 3Dong Seongbuk ku, Seoul(KR)**

(74) Representative: **Thévenet, Jean-Bruno et al Cabinet BEAU DE LOMENIE 55 rue d'Amsterdam**
**F-75008 Paris(FR)**

(54) **Pulse-taking diagnostic apparatus.**

(57) A pulse-taking diagnostic apparatus includes a pressure sensor (4) which is provided with air tubes (1a, 1b, 1c) defining three hermetically sealed spaces making substantially the round of the finger (9) of a hand when the apparatus is mounted in position, and with a pressure sensor (3a, 3b, 3c) provided on one end of each tube for measuring pressure changes in the air tube. The apparatus also includes a blood flow control device adapted to apply a pressure from outside the pressure sensor (4) to control the blood flow when the apparatus is attached to the finger (9).

**FIG.2**

## Pulse-Taking Diagnostic Apparatus

BACKGROUND OF THE INVENTION

Field of the Invention

This invention relates to a pulsatory (pulse-taking) diagnostic apparatus for diagnosing blood pulse.

Description of the Prior Art

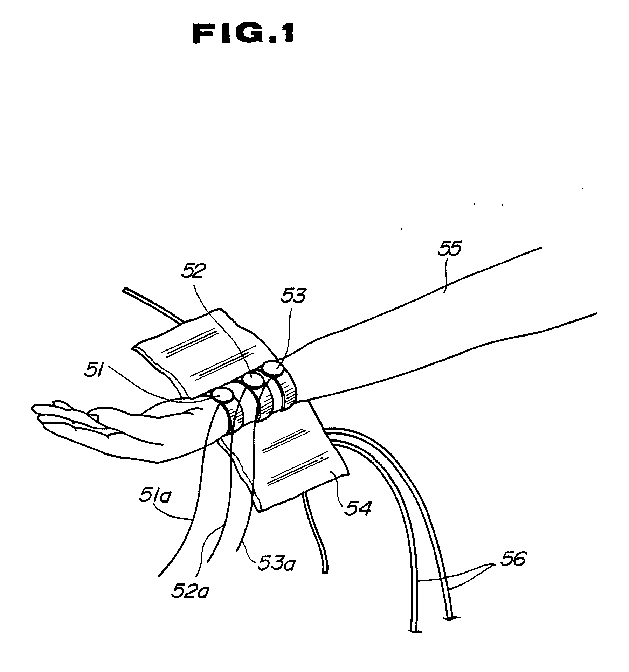

As the conventional pulsatory diagnostic apparatus, the Japanese Patent Publication No. 57-52054 (1982) teaches a pulsatory diagnostic apparatus for Chinese medical science which is herein shown in Fig. 1.

The apparatus shown in Fig. 1 includes three crystal microphones 51, 52, 53, each consisting of a piezo-electric unit, and a tightening cloth 54 for pressing and securing the crystal microphones 51, 52, 53 to the wrist artery against accidental movements.

The piezo-electric microphones 51 to 53 are placed in juxtaposition on the wrist artery and the cloth 54 is wound and attached around the microphones. Air is introduced into an air pouch, not shown, provided within the interior of the tightening cloth 54, by way of an air pump, also not shown. The amount of air thus introduced into the air pouch is adjusted to vary the pressure applied to the artery to measure the changes between the high and low pulse values.

The three piezoelectric microphones are used for measuring the three different pulses "sun", "kan" and "shaku" in terms of the chinese medical science. The "sun", "kan" and "shaku" mean the pulses at the distal side, at the intermediate site and at the heart side of the artery. The pulse of the "sun" measured by piezo-electric microphone 51 represents the state of health from the head to the breast, the pulse of "kan" measured by the piezo-electric microphone 52 represents the state of health from the breast to the navel, and the pulse of the "shaku" measured by the piezo-electric microphone 53 represents the state of health from the navel to the toe, so that the state of health of the human body as a whole may be grasped by measuring the three pulses at the "sun", "kan" and "shaku".

The piezo-electric microphones 51, 52, 53 are connected via connecting cords 51a, 52a, 53a respectively, to an electro-magnetic oscillograph for recording the measured pulses on a recording paper or the like.

However, with the conventional pulse-taking diagnostic apparatus, it has been necessary to locate the artery at which to take the pulse, to apply the piezo-electric microphones 51, 52, 53 accurately to the artery and secure the microphones by the tightening cloth, which necessitates a great deal of skill on the part of an operator. There is also raised a problem that, depending on the mode of tightening of the tightening cloth, the artery may be moved to and fro to render it impossible to procure accurate data.

In addition, most of the pulse-taking diagnostic apparatus are designed to be attached to the person's arm or wrist, so that the apparatus tends to be bulky in size and inconvenient in transport or storage.

OBJECT AND SUMMARY OF THE INVENTION

It is therefore a principal object of the present invention to provide a pulse-taking diagnostic apparatus which may be attached easily, may measure the pulse accurately and may be transported or stored easily because of small size.

According to the present invention, provided a there is pulse-taking diagnostic apparatus comprising pressure detection means provided in three hermetically sealed spaces formed by, for example, flexible tubes substantially making the round of the finger of a hand of a person to be diagnosed and including pressure microphones which are pressure sensors for sensing pressure changes in said hermetically sealed spaces, and a tightening cloth as blood flow control means for controlling the blood flow by applying a pressure from outside of the pressure sensors with said pressure sensors attached to the finger of the person being diagnosed.

With the pulse-taking diagnostic apparatus of the present invention, the flexible tubes are placed around the finger of a hand, and the pulse sounds propagated within the spaces of the flexible tubes are sensed by piezo-electric microphones, so that the operation of locating the artery of the finger of a hand and accurately placing the piezo-electric microphones, such as pressure sensors, may be dispensed with to enable the pulse to be measured more easily. More specifically, since it suffices to place three hermetically sealed flexible tubes, such as rubber tubes, around the finger of a hand and apply a pressure thereto, the laborious operation of placing the pressure sensor on the artery may be dispensed with to enable the pulse to be measured easily and accurately. Since the pulse may be measured easily from the finger, the apparatus may be reduced in size and transported or stored easily.

BRIEF DESCRIPTION OF THE DRAWINGS

Fig. 1 is a perspective view showing the state of use of a conventional pulse-taking diagnostic apparatus.

Fig. 2 is a perspective view showing a construction of a pulse-taking diagnostic apparatus embodiment the present invention.

Fig. 3 is a perspective view showing the state of use of the pulse-taking diagnostic apparatus of Fig. 2.

Fig. 4 is a block circuit diagram of the pulse-taking diagnostic apparatus.

Fig. 5 shows a waveform of a pulse measured by the apparatus shown in Fig. 2.

DESCRIPTION OF THE PREFERRED EMBODIMENT

By referring to the drawings, a pulse-taking diagnostic apparatus embodying the present invention will be explained in detail.

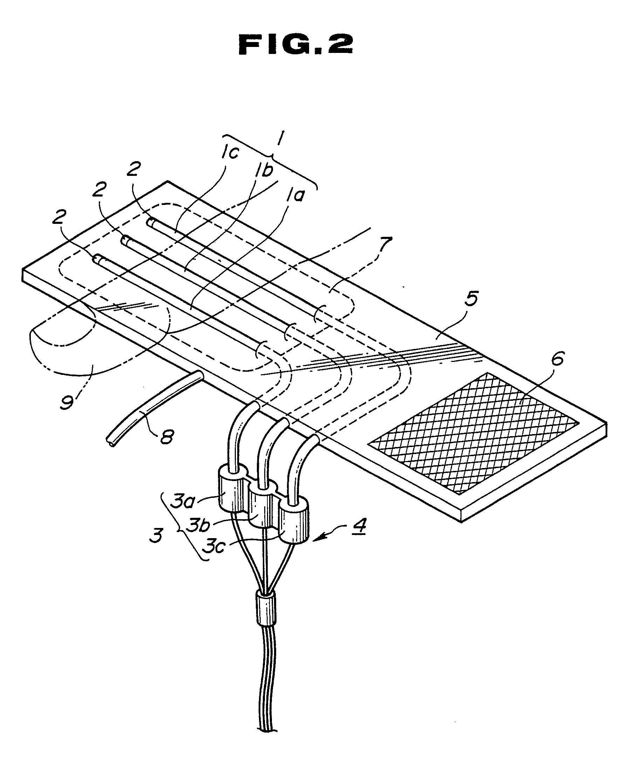

Fig. 2 is a perspective view showing an exemplary construction of a pulse-taking diagnostic apparatus embodying the present invention. With the diagnostic apparatus of the present invention, pulse are measured at the finger of a hand because the same values of the measured pulse data may be obtained from any bodily site of a person being diagnosed.

Referring to Fig. 2, the pulse-taking diagnostic apparatus according to the present invention is generally made up of pressure sensing means 4 composed of three flexible tubes 1a, 1b, 1c of resilient materials, such as rubber, and a tightening cloth 5 in which the resilient tubes 1a to 1c are provided in position, wherein the distal ends of the tubes 1a, 1b, 1c are stopped with plugs 2 against leakage of air trapped in the tubes and the proximal ends of the tubes are connected to three pressure sensors 3a, 3b, 3c, which are conventional omnidirectional microphones, such as capacitor microphones.

Each of the flexible tubes 1a, 1b, 1c is of a length such that each tube makes the round of a finger of a hand 9 when the apparatus is wound and placed around the finger of a hand 9. The tubes 1a to 1c are provided in the tightening cloth 5 so as not to overlap with each other. The tubes 1a, 1b, 1c are placed at the distal side, the mid site and the heart side of the finger 9 for measuring the pulses of "sun" or distal side, "kan" or mid site and "shaku" or heart side, in terms of the Chinese medical science, respectively.

The tightening cloth 5 is provided with fasteners 6, such as so-called magic tapes, so that the cloth 5 may clamp the finger 9 with desired degree of tighteness when the diagnostic apparatus is applied around the finger 9. An air pouch 7 is enclosed in the interior of the cloth 5 so that air from an air pump, not shown, may be pumped via air tube 8 into the pouch 7 to apply a pressure to the flexible tubes 1a, 1b, 1c and the finger 9 to control the blood flow thereat.

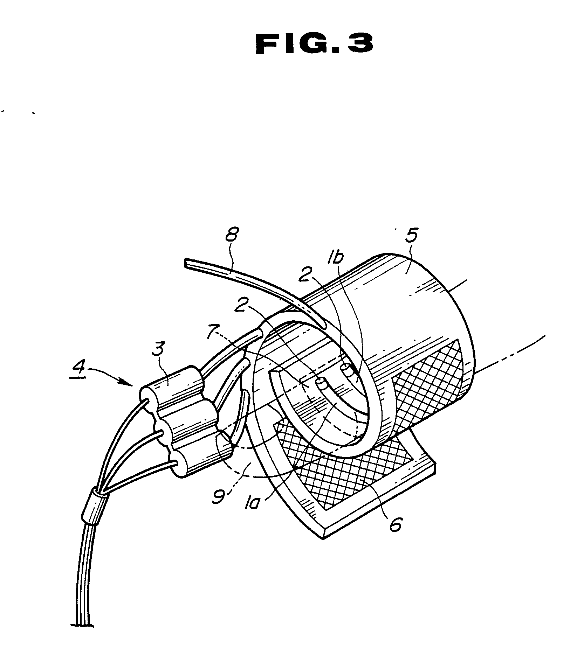

Fig. 3 shows in perspective the state of use of the diagnostic apparatus explained with reference to Fig. 2. In Fig. 3, the same numerals as those in Fig. 2 are used to depict the same or equivalent parts.

Referring to Fig. 3, the flexible tubes 1a, 1b, 1c are placed about the finger 9, along with the tightening cloth 5, in the sequence of the "sun", "kan" and "shaku" as described hereinabove.

Air is introduced via air tube 8 into air pouch 7 enclosed in the tightening cloth 5. When air is introduced into the air pouch 7, the flexible tubes 1a, 1b, 1c are applied under pressure against the finger of a hand 9. Due to the blood vein of the artery of the finger 9, the pressure in the tubes 1a, 1b, 1c is changed. Such pressure change is detected as pulse wave signals for "sun", "kan" and "shaku" by pressure sensors 3a, 3b, 3c, respectively.

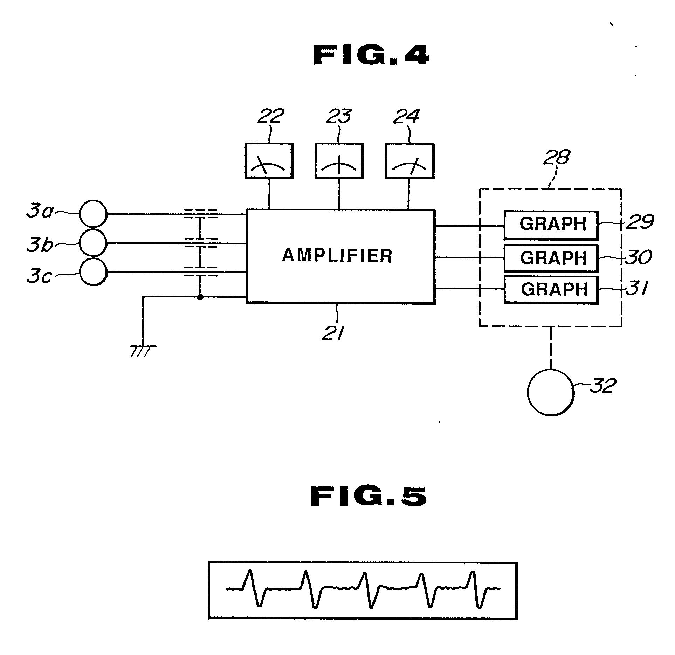

Fig. 4 is a circuit block diagram of the above described embodiment of the pulse-taking diagnostic apparatus of the present invention. In Fig. 4, the same numerals as those used in Figs. 2 and 3 are used to depict the same or equivalent parts or components.

Referring to Fig. 4, the pulse wave signals for "sun", „kan' and "shaku", thus detected by the pressure sensors 3a, 3b, 3c, are amplified by an amplifier 21 and input to ammeters 22, 23, 24 via special filters, not shown, provided in the amplifier 21 to take out pulse components in the signal, while being also input to graphs 29, 30, 31, such as electro magnetic oscillographs, enclosed in a recorder 28.

The recorder 28 is rotated in unison with the graphs 29, 30, 31 by an electric motor 32 for recording the pulse wave signals for "sun", "kan" and "shaku" from the special filters on a recording paper, for example, with recording pens provided in the graphs 29, 30, 31, as shown in Fig. 5.

According to the Chinese medical science, the pulse for "sun" represents the state of health from head to breast, that for "kan" represents the state of health from breast to navel and that for "shaku" represents the state of health from navel to toe. If any defective site is present in a body, one of the pulse wave signals for "sun", "kan" or "shaku" is recorded only with weak amplitude or not recorded whatsoever, so that the state of the bodily health may be recognized at a glance.

In the above described pulse-taking diagnostic apparatus of the present invention, three flexible tubes are used independently. Alternatively, the three flexible tubes may be in communication with one another so that the same pressure may be applied to the three flexible tubes to produce pulse waves accurately.

It will be appreciated from the foregoing that the pulses for "sun", "kan" and "shaku" may be detected accurately from the artery in the finger by the pulse-taking diagnostic apparatus applied around the finger. Since the apparatus is applied to the finger of a hand, it may be reduced in size and manufacture costs and transported or stored easily.

The thus detected pulses for "sun", "kan" and "shaku" may be freely interpreted and applied to clinical tests by taking advantage of the principles of five rows of yin and yang of the oriental medical science and the basic types of the pulse waves.

The six pulse types, that is, acute, slow, large, small, smooth and gradual are subdivided into stronger and weaker to define 12 basic pulse types, which are further subdivided in light of the yin and yang, that is five viscera and six entrails (vicissitudes) into 24 types. These may be considered in light of the left and right side "sun", "kan" and "shaku" sites to define 144 basic subtypes.

It is to be noted that there is sufficient room for evolving numerous diagnostic methods by the application of the present invention to the art of pulse-taking diagnosis.

## Claims

1. A pulse-taking diagnostic apparatus comprising pressure detection means (4) including means (1a, 1b, 1c) forming three hermetically sealed spaces adapted for making substantially the round of a finger (9) of a hand when the apparatus is attached in position and pressure sensors (3a, 3b, 3c) provided in said space forming means (1a, 1b, 1c) for detecting pressure changes in said spaces, and blood flow controlling means for applying pressure from outside of said pressure detection means (4) attached in position for thereby controlling the blood flow.

2. The apparatus according to claim 1 characterized in that said blood flow control means include a tightening cloth (5) and air pouch (7) which is provided in said tightening cloth (5) and into which air is introduced from outside.

3. The apparatus according to claim 2, characterized in that said blood flow control means further include fastening means (5).

4. The apparatus according to claim 1, characterized in that said means (1a, 1b, 1c) for defining the above three hermetically sealed spaces are provided in said blood flow control means so as not to overlap with each other when the apparatus is attached to the finger.

5. The apparatus according to claim 4, characterized in that said means (1a, 1b, 1c) for defining the three hermetically sealed spaces consist of three air tubes which are provided in the longitudinal direction of said blood flow control means.

6. The apparatus according to claim 5, characterized in that said three air tubes are provided in said blood flow control means so that said air tubes are spaced from one another on substantially the same axis when the apparatus is attached in position on the finger (9).

# FIG.1

# FIG.2

# FIG. 3

# FIG.4

# FIG.5

EP 0 395 519 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 066 066 (HEE SOO PAEK) * column 1, lines 7-36; column 4, lines 30-61; figures 6,7 * | 1 | A 61 B 5/022 |
| A | | 2-4 | |
| Y | US-A-2 826 191 (B.D. BURNS) * column 1, line 55 - column 3, line 22; figures 1,2 * | 1 | |
| A | | 2,3 | |
| A | US-A-3 156 237 (K.W. EDMARK) * column 1, line 58 - column 2, line 67; figure 4 * | 1,2,4,5 | |

TECHNICAL FIELDS
SEARCHED (Int. Cl.5)

A 61 B 5/00

The present search report has been drawn up for all claims

| Place of search | Date of completion of the search | Examiner |
|---|---|---|
| BERLIN | 24-07-1990 | WEIHS J.A. |